# EUROPEAN PATENT APPLICATION

(11) **EP 3 047 849 A1**
(43) Date of publication of application: **27.07.2016**
(21) Application number: 14846442.3
(22) Date of filing: 17.09.2014
(51) Int. Cl.: A61K 31/22, A61P 7/00

(54) **COMPOSITION FOR PROMOTING DIFFERENTIATION OR PROLIFERATION OF ERYTHROCYTIC CELLS, CONTAINING MONOACETYL DIACYLGLYCEROL COMPOUND AS ACTIVE INGREDIENT**

(30) Priority: 17.09.2013 KR 20130111890
(71) Applicant: Enzychem Lifesciences Corporation, Daejeon 305-732 (KR); Korea Research Institute of Bioscience and Biotechnology, Daejeon 305-806 (KR)
(72) Inventor: KIM, Jae Wha, Daejeon 305-806 (KR); JUNG, Hai Young, Daejeon 305-806 (KR); KANG, Ho Bum, Daejeon 305-806 (KR); OH, Sei-Ryang, Daejeon 305-806 (KR); AHN, Kyung Seop, Daejeon 305-806 (KR); LEE, Tae-Suk, Daejeon 302-847 (KR); KANG, JongKoo, Cheongju-si Chungcheongbuk-do 362-230 (KR); KIM, Myung Hwan, Seoul 138-736 (KR); HAN, Yong-Hae, Seoul 133-819 (KR); SOHN, Ki Young, Seoul 104-803 (KR)
(74) Representative: Fiammenghi, Eva
(86) International application number: PCT/KR2014/008647
(87) International publication number: WO 2015/041458

(57) **Abstract**

The present invention relates to compositions for promoting differentiation or proliferation of erythrocytic cells containing a monoacetyl diacylglycerol compound as an active ingredient, and pharmaceutical compositions and health functional food compositions for preventing, treating or alleviating erythrocytopenia containing the same. The monoacetyldiacylglycerol compound of the present invention is effective in promoting differentiation or proliferation of erythrocytic cells. The compound can overcome the side effects of the conventional anemia treatment medicines, and has less toxicity. Thus, the compound can be effectively used for preventing, treating or alleviating oligocythemia.

## Description

### [Technical Field]

The present invention relates to compositions for promoting differentiation or proliferation of erythrocytic cells containing a monoacetyl diacylglycerol compound as an active ingredient, and pharmaceutical compositions and health functional food compositions for preventing, treating or alleviating erythrocytopenia containing the same.

### [Background Art]

Anemia is one of erythrocytopenia symptoms, and is defined as a physiological response of a reduction of oxygen supply to tissue cells due to a decrease of the number of hemoglobins, a decrease of the amounts of hemoglobins in blood, or a decrease of oxygen affinity of hemoglobin molecules. There are over 400 kinds of anemia, and the anemia occurs by various reasons such as iron deficiency, deficiency of nutrients such as folic acid, hemolytic or inflammatory disease, bone marrow suppression and so on. It is reported that the anemia occurs very frequently, and more frequently occurs for pregnant women than for men. The frequently occurring anemia accompanies various pathological symptoms, and thus various methods are used to treat the anemia in accordance with the cause of the anemia.

The anemia treatment by supplying iron component or nutrients can be relatively easily performed by a dietary therapy. Meanwhile, when the cause of the anemia relates to proliferation and differentiation of hematopoietic stem cells, it is tried to develop anemia treating medicines by using growth factors, cytokines, dexamethasone, androgens, estrogens and so on. For example, Aranesp, Procrit, nandrolone which is one of anabolic steroids and so on are used to treat the anemia. However, there are some side effects of inducing other diseases. For example, it is known that the anemia treating medicines including erythropoiesis-stimulating agents such as Aranesp and Procrit may increase occurrences of stroke. As other approach, it is recently tried to differentiate into erythrocytes (red blood cells) outside of a body, and supply the differentiated erythrocytes into the body. Namely, CD34+ hematopoietic stem cells is purely separated and cultured and proliferated outside of a body, and then induced to differentiate into erythrocytes by using erythropoietin. However, the processes require many cytokines, which makes the processes economically unfavorable.

EC-18 is a monoacetyldiglyceride and has been separated from deer antler. It has been reported that EC-18 increases survivability ratio of animals in sepsis animal model experiment using cecal-ligation-puncture, and shows no toxicity in a GLP (Good Laboratory Practice) toxicity test. However, the effect of the monoacetyldiacylglycerol compounds including EC-18 on erythrocytopenia such as anemia is not known or disclosed in the prior art.

### [Disclosure]

### [Technical Problem]

The present inventors researched to develop a new agent for preventing or treating erythrocytopenia such as anemia without side effects, and found that monoacetyldiacylglycerol compounds promote the differentiation or proliferation of erythrocytic cells, and can be used to prevent or treat erythrocytopenia, which results in the present invention. An object of the present invention is to provide compositions for promoting the differentiation or proliferation of erythrocytic cells. Other object of the present invention is to provide pharmaceutical compositions or health functional food compositions for preventing, treating or alleviating erythrocytopenia (oligocythemia). Another object of the present invention is to provide methods for preventing or treating oligocythemia.

### [Technical Solution]

In an embodiment for achieving the objects, the present invention provides a composition for promoting differentiation or proliferation of erythrocytic cells, comprising a monoacetyldiacylglycerol compound of Formula 1 as an active ingredient. 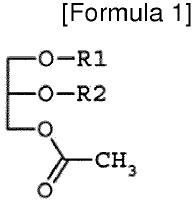 wherein R1 and R2 are independently a fatty acid residue of 14 to 20 carbon atoms.

Also, the present invention provides a pharmaceutical composition or a health functional food composition for preventing, treating or alleviating oligocythemia comprising the monoacetyldiacylglycerol compound of Formula 1 as an active ingredient. Also, the present invention provides a method for preventing or treating oligocythemia, comprising a step of administering the pharmaceutical compound of formula 1 to a non-human or human subject.

The composition for promoting differentiation or proliferation of erythrocytic cells of the present invention comprises a monoacetyldiacylglycerol compound of Formula 1. In the present disclosure, the term "monoacetyldiacylglycerol compound" means a glycerol derivative containing an acetyl group and two acyl groups and is also referred to as monoacetyldiacylglycerol (MADG). In the monoacetyldiacylglycerol compounds of Formula 1, R1 and R2 are independently a fatty acid residue of 14 to 20 carbon atoms, and preferable and non-limiting examples thereof include palmitoyl, oleoyl, linoleoyl, linolenoyl, stearoyl, myristoyl, arachidonoyl, and so on. More preferable and non-limiting combinations of R1 and R2 (R1/R2) include oleoyl/palmitoyl, palmitoyl/oleoyl, palmitoyl/linoleoyl, palmitoyl/linolenoyl, palmitoyl/arachidonoyl, palmitoyl/stearoyl, palmitoyl/palmitoyl, oleoyl/stearoyl, linoleoyl/palmitoyl, linoleoyl/stearoyl, stearoyl/ linoleoyl, stearoyl/oleoyl, myristoyl/linoleoyl, myristoyl/oleoyl, and so on. Stereoisomers of the compound of Formula 1 are also included in the scope of the present invention. In optical activity, the monoacetyldiacylglycerol compounds of Formula 1 can be (R)-form, (S)-form or a racemic mixture.

Preferably, the monoacetyldiacylglycerol compound is a compound of the following Formula 2:

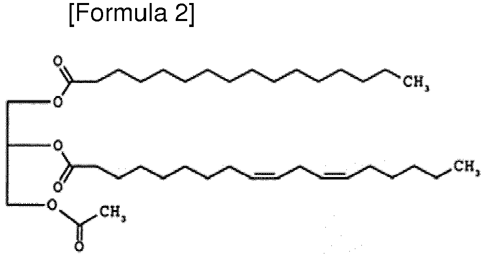

The compound of Formula 2 is 1-palmitoyl-2-linoleoyl-3-acetylglycerol, sometimes referred as "EC-18" or "PLAG". R1 and R2 of the compound correspond to palmitoyl and linoleoyl, respectively.

The monoacetyldiacylglycerol compounds can be separated/extracted from a deer antler or can be produced by known organic synthesis methods. Specifically, deer antler is extracted with hexane, followed by extracting the residue with chloroform and distilling the obtained extracts under a reduced pressure to provide chloroform extracts. The volume of the solvents, hexane and chloroform, for this extraction is just enough to immerse the deer antler. In general, about 4-5 liters of hexane and chloroform for 1 kg of deer antler is used, but not limited thereto. The chloroform extracts of deer antler obtained by this method is further fractionated and purified using series of silica gel column chromatography and TLC method to obtain the monoacetyldiacylglycerol compound for the present invention. An eluent for the chromatography purification process includes chloroform/methanol, hexane/ethylacetate, hexane/ethylacetate/acetic acid, and so on, but not limited thereto. Meanwhile, a chemical synthetic method for the preparation of monoacetyldiacylglycerol compounds of the present invention is shown in, for example, Korean Patents No. 10-0789323. Specifically, the method comprises (a) a step of preparing 1-R1-3- protecting group -glycerol by adding a protecting group in the position 3 of 1-R1-glycerol; (b) a step of preparing 1-R1-2-R2-3-protecting group-glycerol by introducing R2 in the position 2 of the 1-R1-3- protecting group-glycerol; and (c) a step of preparing the desired monoacetyldiacylglycerol compound by performing a deprotection reaction and the acetylation reaction of the 1-R1-3- protecting group -glycerol at the same time. The monoacetyldiacylglycerol compound may be further purified if necessary. Alternatively, monoacetyldiacylglycerol compounds can be prepared by acetolysis of phosphatidylcholine but is not limited thereto.

In the present invention, the term "erythrocytic cell" includes all cells which are matured from erythroid progenitor cell, and means all cells which are under all differentiation processes from erythroid progenitor cell to erythrocyte. More preferably, the erythrocytic cells can be erythroid progenitor cells or erythrocyte cells, but are not limited thereto. In the present specification, the term "erythroid progenitor cell" means all cells under erythrocyte forming process except for completely matured erythrocyte. In bone marrow, there exists hematopoietic stem cell which can be differentiated into various blood corpuscles. Some of the hematopoietic stem cell is differentiated into erythroid progenitor cell which is further differentiated into erythrocyte. The most primitive erythroid progenitor cell is BFU-E(burst forming unit- erythroid), and a slightly differentiated one is CFU-E(colony forming unit-erythroid). After CFU-E, the erythroid progenitor cell is divided and differentiated into proerythroblast, basophilic erythroblast, polychromatophilic erythroblast, orthochromatic erythroblast and so on, and thereafter polychromatic erythrocyte is matured into erythrocyte by enucleation. At the stages of BFU-E and CFU-E, the differentiation into erythrocyte is firmly determined, and BFU-E and CFU-E cannot be differentiated into other blood corpuscles except for erythrocyte. Thus, when the number of BFU-E and CFU-E increases, erythrocyte formation is promoted.

The homeostasis of erythrocyte formation is mainly maintained by erythropoetin(EPO) which is a hematopoiesis factor. Erythropoetin is mainly produced in a kidney, circulates in blood, stimulates CFU-E in bone marrow to differentiate and proliferates CFU-E, and thereby promotes erythrocyte formation. When EPO is not produced to normal level and is deficient, CFU-E and the erythrocyte formation decrease, and erythrocytopenia or anemia may occur. In the present invention, "erythrocyte cell" is a major component in number in blood cells, has a shape of red flat discus, supplies oxygen to tissues of body through blood vessels, removes carbon dioxide from the tissues, and also called as "red blood cell(erythrocyte)". The major function of erythrocyte is to transport oxygen. The erythrocyte includes hemoglobin for transporting oxygen, but does not include nucleus unlike most other cells of body. A normal adult man has 4 to 6 billion erythrocytes in 1 mℓ of blood. The erythrocyte is formed in bone marrow by differentiation of erythroid progenitor cell, and about 2.4 million new erythrocytes are formed in 1 second. The erythrocyte circulates whole body and works generally for 100∼120 days, and obsolete erythrocyte is destroyed by macrophages in reticuloendothelial system organs such as spleen, liver, bone marrow and so on.

In the present invention, the term "differentiation" means a phenomenon in which a cell is grown by division or proliferation to have specialized structures or functions, and namely, means changes of structures or functions of cell, tissue and so on to perform their original functions. Monoacetyldiacylglycerol compound of the present invention promotes the differentiation of hematopoietic stem cell originated from bone marrow into erythrocytic cell. In the present invention, the term "proliferation" means a phenomenon in which a cell is divided into cells having same properties, and generally means a phenomenon in which cells of same type are reproduced to increase the number of the cells. Monoacetyldiacylglycerol compound of the present invention promotes the proliferation of erythrocytic cell.

In Examples of the present invention, TER-119 antibody which is used as a marker of erythrocytic cell is measured by using FACS, which revealed that the bone marrow cells separated from bone marrow of a EC-18 administered mouse includes 41.7% of erythrocytic cells, and the bone marrow cells of a EC-18 non-administered mouse includes 31.2% of erythrocytic cells(Experimental example 1 and Figure 1). Meanwhile, the amounts of other cells originated from bone marrow are maintained same. Thus, it is confirmed that the compound selectively promote the differentiation and proliferation of only erythrocytic cell (Experimental example 2, Figures 2 and 3).

In other embodiment of the present invention, the present invention provides a pharmaceutical composition for preventing or treating oligocythemia comprising the monoacetyldiacylglycerol compound of Formula 1 as an active ingredient. 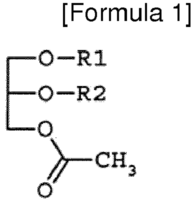 wherein R1 and R2 are independently a fatty acid residue of 14 to 20 carbon atoms.

In the present invention, it is confirmed that the monoacetyldiacylglycerol compound has an activity to promote differentiation or proliferation of erythrocytic cell, and the compound can be effectively used to prevent or treat oligocythemia. Therefore, the pharmaceutical composition of the present invention for preventing or treating oligocythemia comprises the monoacetyldiacylglycerol compound of Formula 1.

In the present invention, the term "oligocythemia" means all diseases in which the number of erythrocytes is less or deficient than that of normal person. Specifically, oligocythemia includes pancytopenia, fanconi's syndrome, granulocytopenia, myelodysplasia or anemia, but is not limited thereto. The causes of oligocythemia are very diverse. Specific examples of the causes include decrease of production of erythropoietin by a kidney failure, erythrocyte production problem in bone marrow such as aplastic anemia, leukemia or cancer at bone marrow, activity decrease of bone marrow stem cell in aged bone marrow, activity decrease of bone marrow cell due to side effects of chemotherapy or radiotherapy for a cancer, and so on, but are not limited thereto. Among them, "anemia" means a phenomenon in which blood does not supply enough oxygen for a metabolism to tissues of a body, and hyposia occurs at the tissue. Since erythrocytes in blood supply oxygen to tissues, the anemia can be diagnosed by the amount of blood pigment (hemoglobin) in erythrocyte. Preferably, the anemia can be aplastic anemia, hemolytic anemia, or iron deficiency anemia, but is not limited thereto.

The monoacetyldiacylglycerol compound, which is an active ingredient of the present invention, promotes differentiation of hematopoietic stem cell originated from bone marrow into erythrocytic cell. Thus, the compound can be used for prevention or treatment of oligocythemia occurred by various causes. In the present invention, the term "prevention" includes any activity to suppress or delay the onset of oligocythemia by administering the composition of the present invention. The term "treatment" includes any activity to improve or advantageously change symptoms of oligocythemia by the composition of the present invention.

The pharmaceutical composition of the present invention may include conventional pharmaceutically acceptable carriers, excipients, or diluents. The amount of monoacetyldiacylglycerol in the pharmaceutical composition can be widely varied without specific limitation, and is specifically 0.0001 to 100 weight%, preferably 0.001 to 50 weight%, and more preferably 0.01 to 20 weight% with respect to the total amount of the composition.

The pharmaceutical composition may be formulated into tablet, bolus, powder, granule, capsule, suspension, emulsion, syrup, sterilized aqueous solution, non-aqueous solution, freeze-dried formulation, suppository, and so on, and can be formulated for oral or non-oral administration. In formulating the composition, conventional excipients or diluents such as fillers, bulking agents, binders, wetting agents, disintegrating agents, and surfactants can be used. The solid formulation for oral administration includes tablet, bolus, powder, granule, capsule and so on, and the solid formulation can be prepared by mixing one or more of the active components and at least one excipient such as starch, calcium carbonate, sucrose, lactose, gelatin, and so on. Besides the excipient, a lubricant such as Magnesium stearate and talc can also be used. The liquid formulation for oral administration includes suspension, emulsion, syrup, and so on, and may include conventional diluents such as water and liquid paraffin or may include various excipients such as wetting agents, sweeting agents, flavoring agents, and preserving agents. The formulation for non-oral administration includes sterilized aqueous solution, non-aqueous solution, freeze-dried formulation, suppository, and so on. Solvent for such solution may include propylene glycol, polyethylene glycol, vegetable oils such as olive oil, and ester for syringe injection such as ethyl oleate. Base materials of the suppository may include witepsol, macrogol, tween 61, cacao butter, Laurin and glycerogelatin.

The compound of the present invention can be administered in a pharmaceutically effective amount. The term "pharmaceutically effective amount" is used to refer to an amount that is sufficient to achieve a desired result in a medical treatment. The "pharmaceutically effective amount" can be determined according to the subject's category, age, sex, severity and type of disease, activity of drug, sensitivity to drug, administration time, administration route, excretion rate, and so forth. The composition of the present invention can be administered alone or with other therapeutic agents sequentially or simultaneously. The composition of the present invention can be administered once or multiple times. The preferable amount of the composition of the present invention can be varied according to the condition and weight of patient, severity of disease, formulation type of drug, administration route and period of treatment. An appropriate total amount of administration per 1 day can be determined by a physician, and is generally 0.001 to 1000 mg/kg, preferably 0.05 to 200 mg/kg, more preferably 0.1 to 100 mg/kg once a day or can be administered in divided doses multiple times daily. The composition of the present invention can be administered to any subject that requires the prevention or treatment of oligocythemia. For example, the composition of the present invention can be administered to not only human but also non-human animals such as monkey, dog, cat, rabbit, guinea pig, rat, mouse, cow, sheep, pig, goat, and so on. The composition of the present invention can be administered by any conventional method, for example, by oral or rectum administration, or by intravenous, intramuscular, subcutaneous, intrauterine dural or cerebrovascular injection.

In another embodiments of the present invention, the present invention provides a health functional food composition for preventing or alleviating oligocythemia, which comprises a monoacetyldiacylglycerol compound of formula 1 as an active ingredient: 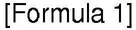 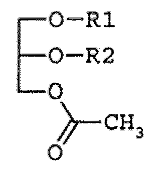 wherein R1 and R2 are independently a fatty acid residue of 14 to 20 carbon atoms, but are not limited thereto.

Since the monoacetyldiacylglycerol compound of the invention promotes the differentiation or proliferation of erythrocytic cells, the compound can be included in health functional food compositions for preventing or improving oligocythemia. The monoacetyldiacylglycerol and oligocythemia are the same as explained above. In the present invention, the term "improvement" includes any activity to improve or ameliorate the symptoms of oligocythemia by administering the composition of the present invention. When the composition of the present invention is included in the health functional food, the composition can be added alone or can be added with other health functional food or other conventional ingredients of health functional food. The amount of the active ingredient can be determined suitably according to the intended use. Generally, when preparing food or beverage, the amount of the composition of the present invention is added preferably in the amount of less than 15 weight part, more preferably less than 10 weight part with respect to the amount of raw material 100 weight part. However, the amount may be increased or decreased. In case of a long term use for the purpose of the health control and hygiene, the amount can be less than the above range. Since there is no problem in terms of safety, the active ingredient may be used in an amount greater than the above range.

Foods to which the compound of the present invention can be added are not limited and include various foods, for example, meats, sausages, breads, chocolates, candies, snacks, pizzas, noodles, gums, daily products such as ice creams, soups, beverages, teas, drinks, alcoholic beverages, vitamin complexes and any health functional food. When the health functional food composition is used in the beverage product, the beverage product may include sweeting agents, flavoring agents or carbohydrates. Examples of carbohydrates include monosaccharides such as glucose and fructose, disaccharides such as maltose and sucrose, polysaccharides such as dextrin and cyclodextrin, and sugar alcohols such as xylitol, sorbitol and erythritol. The amount of carbohydrate in the beverage composition can be widely varied without specific limitation, and is preferably 0.01 to 0.04 g, more preferably 0.02 to 0.03 g per 100 ml of the beverage. Examples of sweeting agents include natural sweeteners such as thaumatin and stevia extract and artificial sweeteners such as saccharin and aspartame. In addition to the above, the health functional food composition of the present invention may include various nutrients, vitamins, electrolytes, flavoring agents, colorants, pectic acid and salts thereof, alginic acid and salts thereof, organic acids, protective colloidal thickening agents, pH controlling agents, stabilizing agents, preserving agents, glycerin, alcohol, carbonizing agents used in carbonated beverages and so on. Moreover, the composition may include fruits, as used in preparing natural fruit juices and fruit juice beverages and vegetable beverages.

In another embodiment of the present invention, the present invention provides a method for preventing or treating oligocythemia, comprising a step of administering the pharmaceutical composition to a subject possibly having oligocythemia. The term "subject possibly having oligocythemia" includes any animal including human that may suffer from oligocythemia or will be suffer from oligocythemia. The subject can be effectively treated by administering the pharmaceutical composition including the compound of the present invention to a subject possibly having oligocythemia. The term "administration" means introducing the pharmaceutical composition of the present invention to a subject by any suitable method. The composition of the present invention can be administered by conventional various methods, for example, by oral or non-oral administration.

The treatment method of the present invention includes administering an effective amount of the pharmaceutical composition comprising the monoacetyldiacylglycerol compound of formula 1. An appropriate total amount of administration per 1 day can be determined by a physician, and is generally 0.001 to 1000 mg/kg, preferably 0.05 to 200 mg/kg, more preferably 0.1 to 100 mg/kg. The total administration amount per day can be administered once a day or can be administered in divided doses multiple times daily. However, the therapeutically effective amount to a specific patient can be varied depending on specific composition, patient's age, body weight, general health status, sex, diet, administration time, administration route, the ratio of composition, treatment period, other drugs used together in the treatment and various factors well known in the medical field.

### [Technical Effects]

The monoacetyldiacylglycerol compound of the present invention is effective in promoting differentiation or proliferation of erythrocytic cells. The compound can overcome the side effects of the conventional medicines used in the treatment of oligocythemia, and has less toxicity. Thus, the compound can be effectively used for preventing, treating or alleviating oligocythemia.

### [Brief Description of the Drawings]

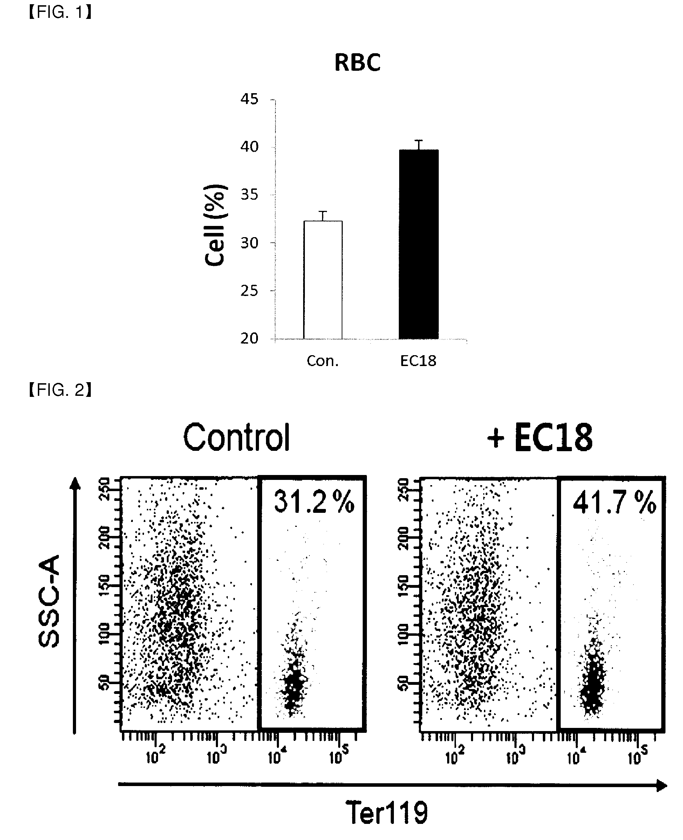

Figure 1 is a graph showing the ratio of erythroid cells in bone marrow cells isolated from mice treated with EC-18 and untreated mice.
Figure 2 is a graph of FACS results showing the ratio of erythroid cells in bone marrow cells isolated from mice treated with EC-18 and untreated mice.

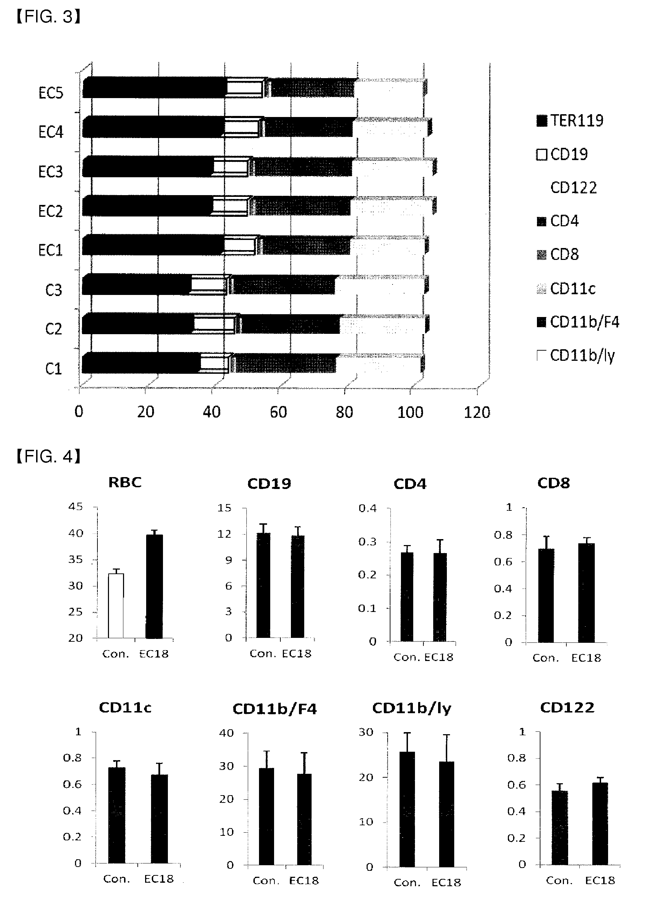

Figure 3 is a graph showing, by comparison, the distribution of erythroid cells, B cells, T cells, dendritic cells, macrophages, neutrophils and NK cells in each mouse of the control group (no treatment group) and the test group (EC-18 treatment group).
Figure 4 is a graph showing, by comparison, differentiation or proliferation of erythroid cells, B cells, T cells, dendritic cells, macrophages, neutrophils and NK cells before and after EC-18 treatment, measured by using TER-119, CD19, CD4, CD8, CD11 c, CD11 b/F4, CD11 b/ly and CD122 antibodies.

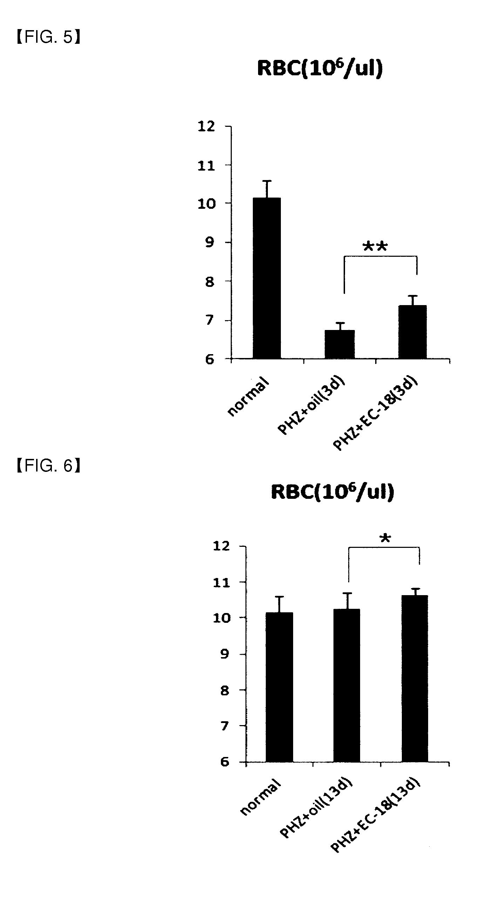

Figure 5 is a graph showing the concentration of erythrocytes 3 days after phenyl hydrazine treatment in the normal group, the control group and the test group.
Figure 6 is a graph showing the concentration of erythrocytes 13 days after phenyl hydrazine treatment in the normal group, the control group and the test group.

### [Examples]

The following examples are provided for better understanding of this invention. However, the present invention is not limited by the examples.

### Experimental example 1: Culture of mouse bone marrow cells

Bone marrow cells isolated from C57BL mouse bone marrow were cultured and maintained in RPMI1640 (Life Technologies, Karlsruhe, Germany) media containing 10% fetal calf serum (FCS, HyClone, Logan, UT), 2 mM L-glutamate, 100 µg/ml penicillin, 100 µg/ml streptomycin (Life Technologies). The cells were cultured at 37 °C under 5% CO₂ humid conditions.

### Example 2: Isolation of bone marrow cells from mice treated with EC-18

Bone marrow cells were isolated from C57BL mice in the following manner. A total of eight 9-week-old C57 BL mice weighting 25-30 mg were used in the experiment. Three mice were used as control (C1-3). EC-18 was administered orally to five mice at a dose of 50 mg/kg for seven days. Mice were sacrificed by tibia dislocation and sterilized with 70% alcohol. The tissue of tibia and femur was cut, skin was removed and muscles were taken out. After removing tibia by breaking it in the opposite direction, the sample was washed with 70% alcohol and placed in PBS. After removing the cartilage between femur and tibia, muscles were removed from femur. Femur was separated from pelvis, washed in 70% alcohol and placed in PBS. In order to isolate bone marrow cells from femur, the pelvic end was cut and after filling the 1 ml syringe with culture media, the needle was injected into the cartilage in the knee side to allow bone marrow cells to be pushed out. The culture media containing bone marrow cells was transferred to a tube and the tube was centrifuged. After discarding the supernatant, bone marrow cells were collected in RPMI 1640 media for flow cytometry.

### Example 3: Analysis of erythroid cells after EC-18 treatment by FACscan

Differentiation and proliferation of erythroid cells in bone marrow cells collected from C57BL mice treated with EC-18 were analyzed by using TER-119 antibody (BD Biosciences-Pharmingen, San Jose, CA, USA), which is a marker of erythroid cells, and FACS. Specifically, FACS was performed as follows. Bone marrow cells isolated by the method of Example 2 were stained by incubating with anti-TER119-FITC antibody and analyzed by using FACscan flow cytometer (Becton Dickinson, San Jose, CA). The results show that the percentage of erythroid cells in bone marrow cells isolated from mice treated with EC-18 (41.7%) is increased, compared to untreated mice (31.2%) (Figure 1 and 2).

### Example 4: Analysis of bone marrow cells by using FACscan

Bone marrow cells isolated from C57BL mice treated with EC-18 (EC1-EC5, EC-18) and untreated mice (C1-C3, con) were compared. TER-119 antibody was used to detect Erythroid cells, CD19 antibody was used to detect B cell, CD4 and CD8 antibodies were used to detect T cells, CD11c antibody was used to detect DC cells, CD11 b/F4 antibody was used to detect macrophages, CD11 b/ly antibody was used to detect neutrophils, and CD122 antibody was used to detect NK cells. Cells were incubated with TER-119, CD19, CD4, CD8, CD11c, CD11b/ly, CD122 antibodies (BD Biosciences-Pharmingen, San Jose, CA, USA) for 1 hour and analyzed using FACscan flow cytometer (Becton Dickinson, San Jose, CA). The results show that the ratio of cells other than erythroid cells in bone marrow cells isolated from mice treated with EC-18 is not changed (Figures 3 and 4).

### Example 5: Analysis of the concentration of erythrocytes in blood

To observe changes in the level of erythrocytes in mouse blood, anemia-induced mice were prepared by administering phenyl hydrazine (PHZ) intraperitoneally into mice weighing 25 mg at a dose of 100 µg/mouse. After administering EC-18 orally to the mice at a dose of 5 mg/ml, blood was collected after 3 days and 13 days. For comparison, a normal group that was not treated with phenyl hydrazine and a control group that was treated with olive oil instead of EC-18 were prepared and blood was collected from the groups after 3 days and 13 days. Collected blood was transferred to EDTA bottle 0.5 ml (Minicollect tube, Greiner bio-one, Austria) and the concentration of erythrocytes (number per µl, unit: 10⁶) was measured using a flow cytometer, Auto Hematology Analyzer BC-5300 (Mindray, Shenzhen, China). The results are shown in Figures 5 and 6. Figure 5 is a graph showing the concentration of erythrocytes 3 days after phenyl hydrazine treatment in the normal group, the control group (treated with PHZ and olive oil) and the test group (treated with PHZ and EC-18). Figure 6 is a graph showing the concentration of erythrocytes 13 days after phenyl hydrazine treatment in the normal group, the control group and the test group. As shown in Figure 5, the concentration of erythrocytes is significantly decreased 3 days after PHZ treatment, whereas EC-18 treatment mitigates the reduction of erythrocytes. Figure 6 shows that the concentration of erythrocytes recovers to the normal level in the control group, whereas the concentration of erythrocytes in the test group is slightly higher than the normal level.

From the above description, a person skilled in the art will appreciate that the invention may be embodied in other specific forms without changing the technical spirit or essential characteristics. In this regard, the examples described above are intended to be illustrative in all respects and it should be understood as not limiting. The scope of the invention should be understood to include all ranges of the above detailed description and the appended claims, rather than the scopes of the specific examples, as well as all such modifications derived from those equivalents.

### [Industrial applicability]

Monoacetyldiacylglycerol compound of the present invention can be effectively used as a composition for preventing, treating or alleviating oligocythemia.

## Claims

1. A composition for promoting differentiation or proliferation of erythrocytic cells, comprising a monoacetyldiacylglycerol compound of Formula 1 as an active ingredient: 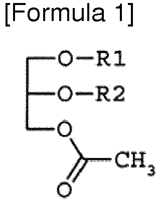 wherein R1 and R2 are independently a fatty acid residue of 14 to 20 carbon atoms.

2. The composition of claim 1, wherein R1 and R2 are independently selected from the group consisting of palmitoyl, oleoyl, linoleoyl, linolenoyl, stearoyl, myristoyl, and arachidonoyl.

3. The composition of claim 1, wherein R1 and R2 (R1/R2) is selected from the group consisting of oleoyl/palmitoyl, palmitoyl/oleoyl, palmitoyl/linoleoyl, palmitoyl/linolenoyl, palmitoyl/arachidonoyl, palmitoyl/stearoyl, palmitoyl/palmitoyl, oleoyl/stearoyl, linoleoyl/palmitoyl, linoleoyl/stearoyl, stearoyl/linoleoyl, stearoyl/oleoyl, myristoyl/linoleoyl and myristoyl/oleoyl.

4. The composition of claim 1, wherein the monoacetyldiacylglycerol compound is a compound of Formula 2:

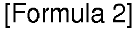

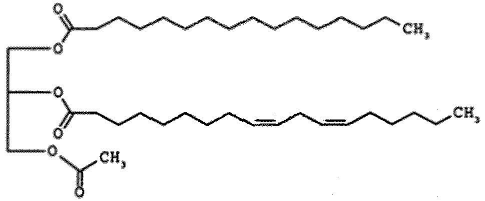

5. The composition of claim 1, wherein the monoacetyldiacylglycerol compound is separated from deer antler.

6. The composition of claim 1, wherein the erythrocytic cells are erythroid progenitor cells or erythrocyte cells.

7. The composition of claim 1, wherein the composition comprises the monoacetyldiacylglycerol compound of formula 1 in an amount of 0.001 to 100 weight%.

8. A pharmaceutical composition for preventing or treating oligocythemia comprising a monoacetyldiacylglycerol compound of Formula 1 as an active ingredient: 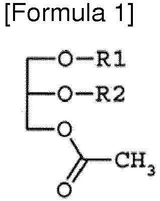 wherein R1 and R2 are independently a fatty acid residue of 14 to 20 carbon atoms.

9. A health functional food composition for preventing or alleviating oligocythemia comprising a monoacetyldiacylglycerol compound of formula 1 as an active ingredient: 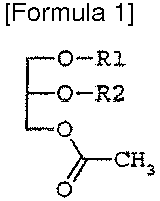 wherein R1 and R2 are independently a fatty acid residue of 14 to 20 carbon atoms.

10. The composition of claim 8 or claim 9, wherein R1 and R2 are independently selected from the group consisting of palmitoyl, oleoyl, linoleoyl, linolenoyl, stearoyl, myristoyl, and arachidonoyl.

11. The composition of claim 8 or claim 9, wherein R1 and R2 (R1/R2) is selected from the group consisting of oleoyl/palmitoyl, palmitoyl/oleoyl, palmitoyl/linoleoyl, palmitoyl/linolenoyl, palmitoyl/arachidonoyl, palmitoyl/stearoyl, palmitoyl/palmitoyl, oleoyl/stearoyl, linoleoyl/palmitoyl, linoleoyl/stearoyl, stearoyl/linoleoyl, stearoyl/oleoyl, myristoyl/linoleoyl and myristoyl/oleoyl.

12. The composition of claim 8 or claim 9, wherein the monoacetyldiacylglycerol compound is a compound of Formula 2:

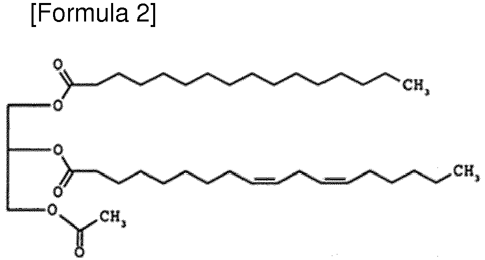

13. The composition of claim 8 or claim 9, wherein the monoacetyldiacylglycerol compound is separated from deer antler.

14. The composition of claim 8 or claim 9, wherein the oligocythemia is pancytopenia, fanconi's syndrome, granulocytopenia, myelodysplasia, aplastic anemia, hemolytic anemia, or iron deficiency anemia.

15. A method for preventing or treating oligocythemia, comprising a step of administering the composition of claim 8 to a non-human subject.

16. The method for preventing or treating oligocythemia of claim 15, wherein the oligocythemia is pancytopenia, fanconi's syndrome, granulocytopenia, myelodysplasia, aplastic anemia, hemolytic anemia, or iron deficiency anemia.
